# FASCICULE DE BREVET EUROPEEN

(11) **EP 0 432 243 B1**
(45) Date de publication et mention de la délivrance du brevet: **23.06.1993**
(21) Numéro de dépôt: 90909614.1
(22) Date de dépôt: 04.07.1990
(51) Int. Cl.: A61K 9/51, A61K 9/127

(54) **PROCEDE DE PRODUCTION DIRECTE DE NANOCAPSULES**
VERFAHREN ZUR DIREKTEN HERSTELLUNG VON NANOKAPSELN
METHOD FOR DIRECT PRODUCTION OF NANOCAPSULES

(30) Priorité: 05.07.1989 FR 8909275
(43) Date de publication de la demande: 19.06.1991
(73) Titulaire: PATRINOVE, F-69006 Lyon (FR)
(72) Inventeur: GUTIERREZ, Gilles, F-69006 Lyon (FR)
(74) Mandataire: Micheli & Cie
(86) Numéro de dépôt international: CH9000162
(87) Numéro de publication internationale: WO9100086

(56) Documents cités:
- EP-A- 0 107 559
- EP-A- 0 190 050
- WO-A-89/02814
- US-A- 4 452 747

## Description

La présente invention concerne la préparation, obtention ou production de nanocapsules incorporant une substance active, en particulier des liposomes.

Les liposomes sont des vésicules, ou capsules, ou sphères artificielles, ayant un diamètre de l'ordre d'une cen-taine de nanomètres, bien connus dans différents domaines techniques, notamment la pharmacie. Ces liposomes sont mis en oeuvre pour incorporer une substance active, soit dans un milieu aqueux remplissant le volume intraliposomial, soit dans la paroi du liposome, et transporter la même substance active vers un site où la substance active sera libérée et utilisée à différentes fins.

Par "substance active", au sens de la présente invention, on entend toute matière, reproduit, composé, ou matériau, susceptible d'être encapsulé selon les techniques usuelles de préparation des liposomes, et présentant une utilité ou fonction technique, ou résultat, dans un processus, procédé ou méthode, de nature physique, chimique, physicochimique thérapeutique, pharmacologique, biologique ou biochimique par exemple.

Ressortent de cette définition :
- les principes actifs médicamenteux
- du matériel biologique, tels que des enzymes, ou fragments d'ADN ou ARN
- des produits cosmétiques, nutritifs
- des produits phyto-sanitaires
- des agents de modification de l'aspect, du goût, ou de la texture d'un aliment.

Les liposomes sont maintenant bien connus, et ont été décrits et étudiés, dans quelques ouvrages ou publications de référence, auxquels on se reportera utilement, et parmi lesquels, on peut citer :
- Les liposomes, applications thérapeutiques, de F PUISIEUX et J. DELATTRE, aux Editions Techniques et Documentation - LAVOISIER.
- Les liposomes en biologie cellulaire et pharmacologie, de Patrick MACHY et Lee LESERMAN, aux Editions INSERM.

En particulier, les liposomes ont été classés en différentes catégories, au rang desquelles on peut distinguer :
- les vésicules multilamellaires, en abrégé MLV
- les grandes vésicules unilamellaires, en abrégé LUV
- les petites vésicules unilamellaires, en abrégé SUV.

Les matières premières nécessaires à la préparation ou synthèse de ces liposomes sont essentiellement les suivantes:
a) Un composé amphiphile, constitutif de la paroi du liposome, tel que :
   - les phospholipides, comme la lécithine, les phosphatidylcholines naturelles ou synthétiques, les sphingomyélines, les phosphatidylinositols, les phosphatidylsérines, les lysophosphatidylcholines, les dicétylphosphates,
   - les détergents de synthèse ou naturels.
b) Eventuellement un composé de rigidification de la paroi du liposome, tel que :
   - un composé polycyclique comportant une chaîne ramifiée, tel qu'un stérol, comme le cholestérol, les phytostérols, la fluorescéine acide et ses esters,
   - certaines amines aromatiques,
   - certains sucres, comme les polyoses, la streptomycine.
c) Eventuellement un composé conférant une charge positive ou négative à la surface de la membrane des liposomes, tel que :
   - le dipalmitoyl phosphatidylglycérol, pour obtenir une charge négative,
   - la stéarylamine, pour obtenir une charge positive.
d) Eventuellement un agent antioxydant du composé amphiphile, et/ou du composé de rigidification, tel que le DL-α-tocophérol.
e) La substance active pouvant être solubilisée en milieu aqueux, ou dans le composé amphiphile, ou dans un milieu organique approprié.

Ces différentes matières sont solubilisées ou mises en suspension dans différents milieux, aqueux et/ou organiques, afin de préparer les liposomes selon différentes méthodes ou voies d'obtention faisant intervenir ces milieux.

Sans entrer dans les détails et le classement des différentes méthodes de préparation, la plupart de celles-ci opèrent par étapes.

La première étape consite à former une pseudomembrane cellulaire, et à transformer cette membrane en une vésicule stable.

La deuxième étape consiste à améliorer les liposomes obtenus selon la première étape, en les triant ou sélectionnant par différents moyens physiques, tels que passage dans une presse de FRENCH, un filtre, une sonication par exemple. Cette étape permet notamment de transformer des populations plurimodales de liposomes de différentes tailles, en des liposomes de petites tailles unilamellaires.

La troisième et dernière étape consiste à éliminer les matières premières non utilisées dans le processus d'encapsulation, ainsi que le ou les milieux de solubilisation ou suspension, par exemple par centrifugation ou chromatographie.

Toutes ces techniques présentent en commun un certain nombre d'inconvénients que l'on peut énumérer comme suit :
- leur rendement est globalement faible, aussi bien en ce qui concerne le taux de matière amphiphile consommée dans le processus d'encapsulation, qu'en ce qui concerne le taux de substance active incorporée dans les liposomes; ce rendement relativement faible allonge corrélativement les temps de production,
- elles mettent en oeuvre des solvants organiques de dissolution, suspension ou dispersion, qu'il faut ensuite éliminer; ceci génère des contraintes de sécurité, toxicité par exemple, et passe par des traitements thermiques consommateurs d'énergie,
- les liposomes obtenus présentent souvent une grande dispersion, quant à leur forme finale, et à la répartition de leurs dimensions unitaires.

La présente invention se réfère à une technique de préparation évitant la succession des étapes mentionnées ci-dessus, autorisant en outre la suppression de tout milieu organique de solubilisation, suspension ou dispersion. Dans un tel cas, on parle de production "directe" de nanocapsules.

S'agissant de la production de nanocapsules par voie directe, EP-A-0190 050 décrit un procédé qui consiste à homogénéiser une phase lipidique hydratée dans des conditions de température et pression adéquates. Ce procédé nécessite la mise en oeuvre d'un appareillage relativement complexe, comportant notamment une valve de détente ainsi qu'un système de recyclage de la phase lipidique émulsionnée. Ce procédé met en oeuvre le phénomène de cavitation en régime turbulent.

La présente invention propose une solution nouvelle et originale au problème posé par la production directe de nanocapsules incorporant une substance active. Elle a plus particulièrement pour objet un procédé que l'on peut décrire comme suit:
- on obtient un liquide de préparation par mélange d'au moins un composé amphiphile, d'un milieu aqueux et de la substance active soluble ou dispersable dans le composé amphiphile ou le milieu aqueux,
- on met en mouvement le liquide de préparation sous forme d'une veine liquide dépourvue de relief interne,
- on soumet la veine liquide en mouvement à une alternance de surpressions et dépressions, générant un phénomène de cavitation au sein de ladite veine.

Par dépressions et surpressions, on entend toutes les pressions respectivement inférieure et supérieure à la pression statique du liquide de préparation, notamment un vide partiel pour la dépression. Lorsque le liquide de préparation est en écoulement, il s'agit de pressions locales respectivement inférieure et supérieure à la pression statique de la veine du liquide en mouvement.

Par milieu aqueux, on entend toute phase aqueuse à l'état pur, ou comportant des sels en solution, par exemple, une solution isotonique apyrogène.

Différentes voies physiques peuvent être utilisées pour soumettre le liquide de préparation en mouvement à une succession de surpressions et dépressions.

Selon l'invention, on utilise le phénomène de cavitation, en général nuisible au bon fonctionnement ou aux performances de différents organes ou machines, tels que pompes ou hélices. Mais ici, selon la présente invention, ce phénomène sera volontairement reproduit, maîtrisé et contrôlé, pour aboutir à une alternance répétée de surpressions et dépressions du liquide de préparation. On sait en effet que la cavitation d'un liquide s'analyse comme la génération au sein de la masse dudit liquide, de zones ou espaces locaux, sous dépression ou vide partiel, et de zones ou espaces locaux en surpression.

La littérature technique décrit également de nombreuses voies de cavitation d'un liquide, sans qu'il soit besoin de les reprendre ici toutes. Ainsi, le phénomène de cavitation peut être généré et obtenu par saturation d'un gaz inerte dans le liquide de préparation, et désaturation du même gaz, lequel provoque alors la cavitation recherchée.

L'invention consiste à faire circuler une veine ou un écoulement du liquide de préparation dépourvu de tout relief interne et à soumettre cette veine au phénomène de cavitation selon le processus dit "coup de bélier" déjà bien étudié, par le choix approprié de conditions opératoires ou paramètres mécaniques ou de construction.

Il s'établira ainsi dans la veine un régime permanent de cavitation générant d'une extrémité à l'autre de la veine des oscillations de pression, c'est-à-dire des noeuds de surpression et dépression, ayant une position relativement stable. Et c'est dans les noeuds de dépression que le liquide subit les phénomènes de cavitation.

Un dispositif de production directe de nanocapsules selon l'invention, comprend :
- une cuve de traitement,
- une pompe dont l'entrée d'aspiration communique avec la cuve,
- un circuit de cavitation extérieur à la cuve, communiquant à une extrémité avec la sortie de refoulement de la pompe,
- des moyens pour alimenter la cuve en liquide de préparation et,
- des moyens pour recueillir les nanocapsules obtenues.

Egalement selon l'invention, la pression statique du liquide de préparation en mouvement est ajustée en-dessus du seuil de fusion des composés amphiphiles présents, en particulier des lipides s'agissant de la préparation des liposomes.

Les paramètres opératoires et mécaniques choisis pour l'écoulement du liquide de préparation, soumis aux oscillations de pression, sont corrélés par l'équation suivante : 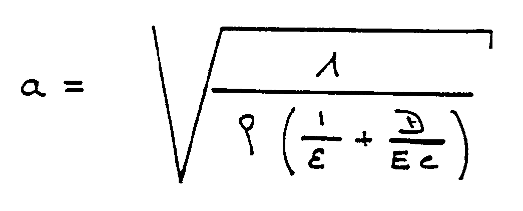
dans laquelle:
a = vitesse de propagation des oscillations de pression, en m/s
ρ = masse volumique du liquide de préparation en Kg/m3
ε = module d'élasticité de volume dudit liquide, en N/m2
D = diamètre de la veine de l'écoulement, en m
e = épaisseur de la paroi canalisant l'écoulement, en m
E = module d'élasticité de ladite paroi en N/m2

Préférentiellement, on choisit selon l'invention de travailler avec une vitesse linéaire d'écoulement au moins égale à 100 m/s dans un conduit à parois lisses et/ou une pression statique du liquide en écoulement au moins égale à 100 bars. De telles conditions permettent, en particulier dans un cricuit fermé, de recycler le liquide de préparation un grand nombre de fois par unité de temps dans les zones de dépression.

Afin de rester en phase aqueuse, on peut préparer un lait de stabilisation par mélange d'un composé de rigidification de la paroi des nanocapsules, tel qu'un composé polycyclique à chaîne dans le cas des liposomes, avec un milieu aqueux de dispersion, et on ajoute ce lait aqueux au liquide de préparation.

Un dispositif de production directe de nanocapsules est maintenant décrit par référence aux dessins annexés dans lesquels :
la figure 1 représente de manière schématique un dispositif selon l'invention,
la figure 2 représente de manière schématique un écoulement du liquide de préparation soumis de manière contrôlée au phénomène de cavitation, et
la figure 3 représente de manière schématique un autre dispositif conforme à l'invention.

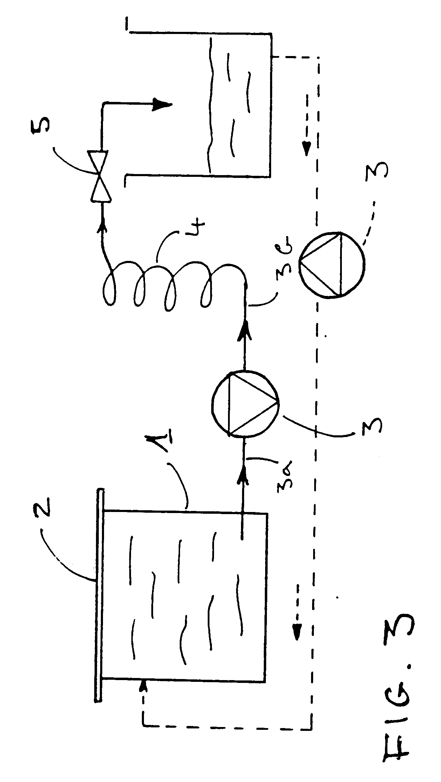

Le dispositif comporte :
- une cuve de traitement (1), résistant à des pressions importantes, par exemple de l'ordre de 100 à 200 bars, pourvue d'un couvercle (2), pouvant être fixé de manière amovible et étanche sur la cuve (1); ce couvercle (2) sert à la fois pour alimenter la cuve en liquide de préparation, et pour évacuer le cas échéant de cette dernière la suspension de nanocapsules obtenue.
- une pompe (3) dont l'entrée d'aspiration (3a) communique avec le fond de la cuve (1),
- un circuit de cavitation (4), extérieur à la cuve (1), communiquant à une extrémité avec la sortie de refoule ment (3b) de la pompe, et à l'autre extrémité, par un détendeur (5), avec une entrée (la) de recyclage dans la cuve (1), au sein de la charge du liquide de préparation. L'opération de recyclage est facultative, un dispositif supprimant cette opération étant illustré par la fig. 3.

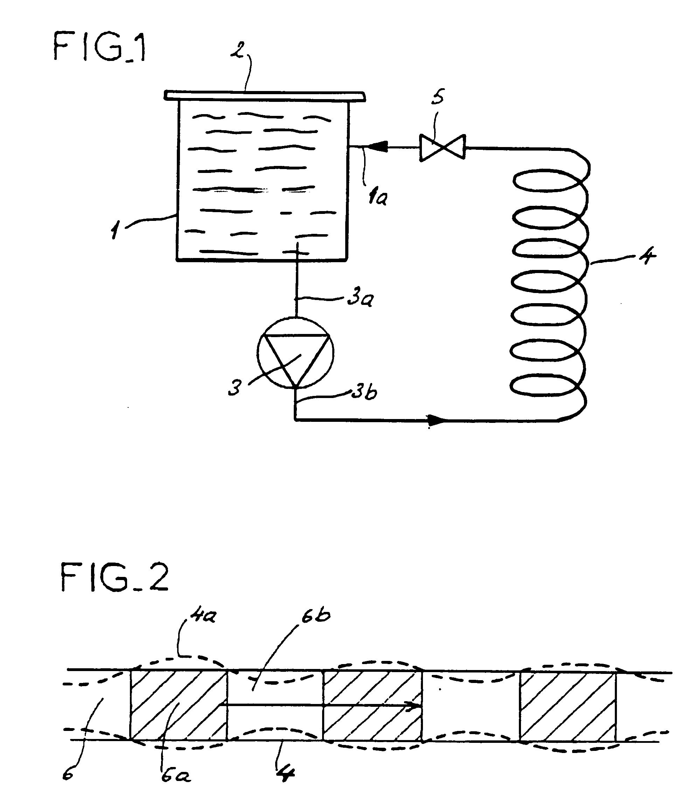

Pour la mise en oeuvre du dispositif précédemment décrit, le couvercle (2) étant ouvert, la cuve (1) est remplie complètement avec le liquide de préparation, obtenu comme décrit précédemment, et éventuellement le lait de stabilisation également discuté précédemment. Puis le couvercle (2) est refermé, de manière à opérer ultérieurement en l'absence de tout air ambiant. La pompe (3) est alors mise en fonctionnement, les différents paramètres opératoires et/ou mécaniques du dispositif selon la figure (1) ayant été préalablement réglés, comme discuté précédemment pour générer et contrôler le phénomène de cavitation dans la boucle (4). Comme montré à la figure 2, dans le circuit de cavitation (4) dessiné de manière droite et développée, on obtient dans la veine (6) de circulation du liquide de préparation, des oscillations de surpression et dépression, respectivement (6a) et (6b), s'établissant selon un régime stationnaire, et pouvant se matérialiser par des bosses et des creux de la paroi (4a) du circuit (4), montrés en trait pointillés à la figure 2, ceci dans le cas où cette paroi (4a) présente une certaine souplesse tout en résistant à la pression. Ce sont dans les noeuds de dépression (6b) que s'effectue l'évaporation du milieux aqueux, et corrélativement la formation des liposomes, et dans les noeuds de surpression (6a) que s'effectue l'incorporation des produits lipophiles.

Conformément à l'invention, un gaz peut être préalablement dissout au sein du liquide de préparation, c'est-à-dire avant sa mise en mouvement.

Certains gaz comme l'hélium par exemple sont peu solubles dans l'eau et génèrent de ce fait plus facilement des bulles. En outre, plus la différence de pression existant entre les noeuds de haute pression (6a) et les noeuds de basse pression (6b) sera importante, plus la taille des bulles ainsi générées sera grande.

Le nombre total de zones de supression et de dépression dans le circuit de cavitation augmente proportionnellement à la longueur dudit circuit : plus ce nombre sera grand, plus l'histogramme de répartition des nanocapsules sera étroit.

Au plan expérimental, on a utilisé un dispositif selon la figure 1, ayant les caractéristiques suivantes :
- une cuve de traitement (1) ayant un volume de 18 litres.
- une pompe (3) ayant un débit nominal de 950 litres/heures et une pression de refoulement de 175 bars.
- un tuyau constituant la boucle (4) ayant une longueur de 8 m, résistant à une pression de 250 bars; le volume du circuit (4) et de la pompe (3) représentant un volume de 2 litres.
   Avec un dispositif tel que précédemment décrit, on a préparé des liposomes de vitamine E et de bleu trypan.

### EXEMPLE 1

S'agissant des liposomes de vitamine E, pour le volume global de 20 litres du dispositif précédemment décrit, on a utilisé les proportions et matières premières suivantes :
- phosphatidylcholine de soja : 1000 g
- cholestérol : 80 g
- DLαtocophérol : 100 g
- acétate de tocophérol (Vitamine E): 100 g
- ascorbate de sodium : 2 g
- eau apyrogène en quantité suffisante pour 20 litres

Les conditions opératoires étaient les suivantes :
- pression de refoulement de la pompe: 150 bars
- vitesse de circulation de la pompe: 600 l/h
- température ambiante.

Au bout de 5 minutes, un prélèvement effectué révèle que 50% de la lécithine n'est pas encore sphérisée, mais ce taux baisse avec le temps, pour aboutir à un rendement pratiquement de 100 % au bout de 40 minutes.

### EXEMPLE 2

S'agissant des liposomes de bleu trypan, on utilise les mêmes conditions opératoires, la vitamine E ou acétate de tocophérol étant remplacée par la même quantité de bleu trypan.

La solution liposomiale obtenue finalement montre que la quantité de colorant encapsulé dans la phase hydrophile est de 46%. On élimine le colorant non encapsulé par chromatographie sur une résine Séphadex G 50. Par élution des liposomes pendant 24 heures, on constate que ces derniers ont gardé 100 % de leur colorant, ce qui démontre leur stabilité et imperméabilité.

En définitive, le procédé selon l'invention permet en outre d'obtenir les avantages significatifs suivants. Tout d'abord, ce procédé s'avère indépendant de la température de transition du composé amphiphile, et on peut donc travailler à toutes températures, au voisinage de la température ambiante par exemple, et par conséquent traiter toutes substances actives sensibles à l'action de la température, par exemple en termes de dégradation.

L'étude physique des nanoparticules, en particulier des liposomes obtenus selon l'invention, montre que leurs dimensions et leurs histogrammes de répartition sont peu sensibles à l'action de la température.

Pour terminer, le phénomène de cavitation permet de travailler uniquement en présence de la vapeur du liquide de préparation, ou celle d'un gaz inerte volontairement dissout, et donc en général en atmosphère inerte, non susceptible d'oxyder les matières premières sensibles à une dégradation par oxydation.

## Revendications

1. Procédé de production directe de nanocapsules incorporant une substance active, caractérisé en ce qu'on obtient un liquide de préparation par mélange d'au moins un composé amphiphile, d'un milieu aqueux et de la substance active, soluble ou dispersable dans le composé amphiphile ou le milieu aqueux, et que l'on met en mouvement le liquide de préparation sous forme d'une veine liquide dépourvue du relief interne, au sein de laquelle on génère un phénomène de cavitation par une alternance de pressions respectivement supérieures et inférieures à la pression statique de la veine de liquide en mouvement.

2. Procédé selon la revendication 1, caractérisé en ce que la pression statique du liquide de préparation en mouvement est ajustée en-dessus du seuil de fusion des éléments amphiphiles présents dans le liquide.

3. Procédé selon revendication 1, caractérisé en ce que les paramètres opératoires et mécaniques choisis pour l'écoulement du liquide de préparation, soumis à des oscillations de pression, sont corrélés par l'équation suivante: 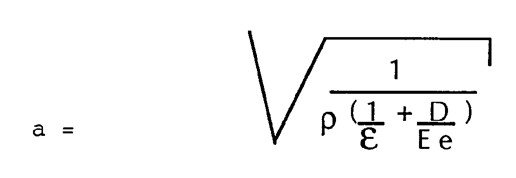 dans laquelle:
a = vitesse de propagation des oscillations de pression, en m/s
ρ = masse volumique du liquide de préparation kg/m³
ε = module d'élasticité de volume dudit liquide, en N/m²
D = diamètre de la veine de l'écoulement, en m
e = épaisseur de la paroi canalisant l'écoulement, en m
E = module d'élasticité de la dite paroi en N/m.

4. Procédé selon la revendication 1, caractérisé en ce que le phénomène de cavitation est obtenu par un écoulement du liquide de préparation ayant une vitesse linéaire d'au moins 100 m/s dans un conduit à paroi lisse.

5. Procédé selon la revendication 1, caractérisé en ce que le liquide de préparation est mis en circulation dans un circuit comprenant une pompe, une cuve de traitement, et un circuit de cavitation extérieur à la cuve.

6. Procédé selon la revendication 5, caractérisé en ce que le circuit est ouvert ou fermé.

7. Procédé selon l'une des revendications précédentes, caractérisé en ce que le liquide de préparation contient un gaz préalablement dissout.

8. Procédé selon l'une des revendications précédentes appliqué à la préparation de liposomes.

## Patentansprüche

1. Verfahren zur direkten Herstellung von Nanokapseln, die eine Aktivsubstanz enthalten, dadurch gekennzeichnet, dass man eine Herstellungsflüssigkeit erhält durch Mischung von zumindest einer amphiphilen Verbindung, eines wasserhaltigen Mediums und der Aktivsubstanz, die in der amphiphilen Verbindung oder im wasserhaltigen Medium löslich oder dispergierbar ist, und dass man die Herstellungsflüssigkeit in Bewegung versetzt in Form eines flüssigen Stromfadens ohne inneres Relief, in welchem man ein Kavitationsphänomen durch Abwechslung von Druckwerten bildet, die respektive höher und tiefer sind als der statische Druck des sich in Bewegung befindenden Stromfadens.

2. Verfahren nach Anspruch 1, dadurch gekennzeichnet, dass der statische Druck der sich in Bewegung befindenden Herstellungsflüssigkeit über der Schmelzgrenze der in der Flüssigkeit vorhandenen amphiphilen Verbindungen eingestellt ist.

3. Verfahren nach Anspruch 1, dadurch gekennzeichnet, dass die für den Fluss der unter Druckschwingungen stehenden Herstellungsflüssigkeit gewählten präparativen und mechanischen Parameter miteinander durch die folgende Gleichung in Verbindung stehen : 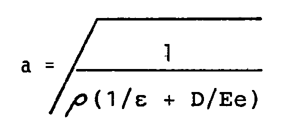 in welcher :
a = Fortpflanzungsgeschwindigkeit der Druckschwingungen in m/s
ρ = spezifische Masse der Herstellungsflüssigkeit kg/m
ε = Volumenelastizitätsmodul der benannten Flüssigkeit, in N/m
D = Durchmesser des Stromfadens, in m
e = Dicke der den Fluss leitenden Wand, in m
E = Elastizitätsmodul der benannten Wand, in N/m.

4. Verfahren nach Anspruch 1, dadurch gekennzeichnet, dass das Kavitationsphänomen durch den Fluss der Herstellungsflüssigkeit mit einer Lineargeschwindigkeit von zumindest 100 m/s in einer mit glatten Wänden versehenen Leitung erhalten wird.

5. Verfahren nach Anspruch 1, dadurch gekennzeichnet, dass die Herstellungsflüssigkeit in einem eine Pumpe, eine Behandlungswanne und einen sich ausserhalb der Wanne befindenden Kavitationsumlauf aufweisenden Kreislauf in Zirkulation gebracht wird.

6. Verfahren nach Anspruch 5, dadurch gekennzeichnet, dass der Kreislauf offen oder gesschlossen ist.

7. Verfahren nach einem der vorhergehenden Ansprüche, dadurch gekennzeichnet, dass die Herstellungsflüssigkeit ein vorher gelöstes Gas enthält.

8. Verfahren nach einem der vorhergehenden Ansprüche, welches zur Herstellung von Liposomen verwendet wird.

## Claims

1. Process for the direct preparation of nanocapsules incorporating an active substance, characterized in that a preparatory liquid is obtained by mixing together at least one amphiphilic compound, an aqueous medium and the active substance, dissolved or dispersed in the amphiphilic compound or in the aqueous medium, and in that the preparatory liquid is set into motion in the form of a stream of liquid having no inside bulges, and in which a cavitation phenomenon is generated by an alternation of pressures respectively higher and lower than the static pressure of the moving liquid stream.

2. Process according to claim 1, characterized in that the static pressure of the preparatory liquid set in motion is adjusted beneath the melting limit of the amphiphilic compounds which are present within the liquid.

3. Process according to claim 1, characterized in that the operational and the mechanical characteristics chosen for the flow of the preparatory liquid subjected to pressure oscillations are dependent upon each other as indicated by the following relation: 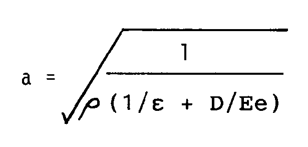 in which :
a = speed of propagation of the pressure oscillations in m/s
ρ = density of the preparatory liquid in kg/m³
ε = bulk modulus of elasticity of said liquid, in N/m²
D = diameter of the stream, in m
e = thickness of the wall channelling the flow, in m
E = modulus of elasticity of said wall in N/m².

4. Process according to claim 1, characterized in that the cavitation phenomenon is obtained by a flow of the preparatory liquid having a linear speed of at least 100 m/s inside a conduit with a smooth wall.

5. Process according to claim 1, characterized in that the preparatory liquid is circulated in a circuit comprising a pump, a treatement tank and a cavitation circuit located outside the tank.

6. Process according to claim 5, characterized in that the circuit is open or closed.

7. Process according to one of the preceding claims, characterized in that the preparatory liquid contains a gas, which was previously dissolved.

8. Process according to one of the preceding claims applied to the preparation of liposomes.
